# DEMANDE DE BREVET EUROPEEN

(11) **EP 1 437 121 A1**
(43) Date de publication de la demande: **14.07.2004**
(21) Numéro de dépôt: 04290757.6
(22) Date de dépôt: 31.05.2000
(51) Int. Cl.: A61K 7/075, A61K 7/06

(54) **Composition de traitement antipelliculaire des cheveux et du cuir chevelu**

(30) Priorité: 08.07.1999 FR 9908877
(62) Demande divisionnaire de: 00401549.1
(71) Demandeur: l'Oreal SA, 75008 Paris (FR)
(72) Inventeur: Maurin, Véronique, 75016 Paris (FR); Beauquey, Bernard, 92110 Clichy (FR)
(74) Mandataire: Le Blainvaux, Françoise

(57) **Abrégé**

La présente invention concerne des compositions cosmétiques ou dermatologiques de lavage et de traitement antipelliculaire des cheveux et du cuir chevelu, comprenant au moins un agent antipelliculaire choisi parmi les sels de pyridinethione, au moins 1% en poids d'au moins un hydroxyacide et au moins un agent tensioactif amphotère.

Ces compositions possèdent une efficacité antipelliculaire améliorée.

## Description

La présente invention concerne des compositions cosmétiques de lavage et de traitement antipelliculaire des cheveux et du cuir chevelu, à base d'agent antipelliculaire, d'hydroxyacide et d'agent tensioactif amphotère.

En vue de combattre la formation des pellicules qui est généralement accompagnée d'une prolifération microbienne et/ou fongique, il a été proposé comme produits anti-pelliculaires soit des produits inhibant la prolifération microbienne, soit des produits kératolytiques. Parmi ces agents antipelliculaires, l'emploi des sels de pyrithione a été tout particulièrement préconisé. De nombreux brevets décrivent les sels de pyridinethione dans les shampooings , par exemple US 3753916, US4345080, US5037818. Les tensioactifs utilisés dans les shampooings sont le plus souvent les tensioactifs anioniques.

Il a également été proposé, dans EP-A-0422508 l'utilisation dans des shampooings, d'agents antibactériens tels que des sels de pyridinethione en association avec un agent tensioactif non ionique du type alkylpolyglucoside.

Cependant, la Demanderesse a constaté que ces shampoings présentaient une activité antifongique en particulier vis-à-vis de Malassezia ovalis encore insuffisante.
De plus, les sels de pyridinethione étant insolubles dans l'eau, ils peuvent également poser des problèmes de mise en suspension s'ils sont présents à des taux relativement importants.

On a maintenant constaté de façon surprenante et inattendue qu'il était possible d'obtenir des compositions notamment cosmétiques de lavage et de traitement antipelliculaire des cheveux et du cuir chevelu présentant une efficacité antipelliculaire améliorée en associant au moins un agent antipelliculaire de type pyridinethione, au moins 1 % en poids d'un hydroxyacide et au moins un agent tensioactif choisi parmi les tensioactifs amphotères.

L'invention a donc pour objet une composition de lavage et de traitement antipelliculaire des cheveux et du cuir chevelu, comprenant dans un milieu aqueux au moins un agent antipelliculaire choisi parmi les sels de pyridinethione, au moins 1% en poids d'au moins un hydroxyacide et au moins un agent tensioactif choisi parmi les tensioactifs amphotères appartenant au groupe des bétaines.

Un autre objet de l'invention est l'utilisation d'une composition telle définie ci-dessus pour le lavage et le traitement antipelliculaire des cheveux et du cuir chevelu.
Un autre objet de l'invention consiste en un procédé de lavage et de traitement cosmétique pour l'élimination des pellicules des cheveux ou du cuir chevelu utilisant ces compositions.

Un autre objet de l'invention est un procédé de traitement de traitement cosmétique ou dermatologique comprenant l'application sur les cheveux ou le cuir chevelu d'une composition telle que définie précédemment, cette application étant suivie d'un rinçage de préférence à l'eau, après un éventuel temps de pose

D'autres objets apparaîtront à la lumière de la description et des exemples qui suivent.

Les sels de pyridinethione sont notamment choisis parmi les sels de calcium, magnésium, barium, strontium, zinc, cadmium, étain et de zirconium. Le sel de zinc est particulièrement préféré.

De tels composés sont notamment commercialisés sous la dénomination Omadine de zinc par la société OLIN.

Le ou les sels de pyridinethione sont présents dans les compositions conformes à l'invention dans des proportions comprises de préférence entre 0,1 et 5% en poids et plus particulièrement entre 0,3 et 2,5% en poids par rapport au poids total de la composition.

Selon une caractéristique essentielle des compositions détergentes selon l'invention, ces dernières contiennent au moins 1% en poids d'un hydroxyacide ou de ses dérivés.

Par dérivés d'acide, on entend ses sels associés (sels avec une base organique ou un alcalin notamment) ou bien encore éventuellement son lactide correspondant (forme obtenu par autoestérification des molécules).

Cet hydroxyacide peut bien entendu consister en un acide mono ou poly carboxylique comportant une ou plusieurs fonctions hydroxy, de préférence l'hydroxyacide est un alpha hydroxy acide : l'une au moins de ces fonctions hydroxy devant occuper une position alpha sur ledit acide (carbone adjacent à une fonction carboxylique). Cet acide peut se présenter dans la composition détergente finale sous forme d'acide libre et/ou sous la forme de l'un de ses sels associés (sels avec une base organique ou un alcalin notamment), en particulier selon le pH final imposé à la composition, ou bien encore éventuellement sous la forme du lactide correspondant (forme obtenu par autoestérification des molécules). Les compositions détergentes conformes à l'invention peuvent bien entendu contenir un ou plusieurs hydroxyacides ou leurs dérivés.

A titre d'exemples de tels composés, on peut citer, entre autres, les acides citrique, lactique, méthyllactique, phényllactique, malique, mandélique, glycolique, tartronique, tartrique, gluconique, benzylique et 2-hydroxycaprylique. D'autres composés de type hydroxyacides convenant à la présente invention sont ceux cités dans la demande de brevet EP-A- 0 413 528, dont l'enseignement est, à cet égard, totalement inclus dans la présente demande.
De préférence, on retiendra les acides qui sont cosmétiquement compatibles et acceptables avec les cheveux, la peau et/ou le cuir chevelu.

Selon un mode particulièrement préféré de réalisation de la présente invention, l'hydroxyacide mis en oeuvre est choisi parmi l'acide citrique, l'acide tartrique et l'acide lactique.

Selon une autre caractéristique importante des compositions détergentes selon l'invention, le ou les hydroxyacides sont présents dans ces dernières à raison d'au moins 1 % en poids, de préférence au moins 2 % en poids par rapport à l'ensemble de la composition. Des teneurs en hydroxyacide(s) comprises entre 2 et 10 % en poids, et plus particulièrement encore entre 2 et 5 % en poids, conviennent généralement très bien. On notera que ces concentrations sont nettement supérieures à celles qui peuvent parfois se rencontrer dans des shampooings de l'art antérieur lorsque certains acides ont été employés uniquement à des fins d'ajustement de pH.

Les agents tensioactifs amphotères peuvent être notamment (liste non limitative) les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Selon la présente invention, on préfère plus particulièrement utiliser les agents tensioactifs amphotères appartenant au groupe des bétaines tels que les alkylbétaïnes en particulier la cocoylbétaïne commercialisée sous la dénomination « DEHYTON® AB 30 » en solution aqueuse à 30 % de MA par la société HENKEL ou les alkyl (C₈-C₂₀) amidoalkyl (C₁-C₆) bétaïnes en particulier la TEGOBETAINE® F 50 commercialisée par la société GOLDSCHMIDT.

Le(s) agent(s) tensioactif(s) amphotère(s) ou non ioniques sont généralement présents à raison de 0,5 à environ 15% en poids, de préférence de 1 à 5% en poids, par rapport au poids total de la composition.

Les compositions de l'invention contiennent en outre avantageusement au moins un autre agent tensioactif qui est généralement présent en une quantité comprise entre 0,1% et 40% en poids environ, de préférence entre 3% et 30% et encore plus préférentiellement entre 5% et 20%, par rapport au poids total de la composition.

Cet agent tensioactif est généralement choisi parmi les agents tensioactifs anioniques.

Les tensioactifs additionnels anionique convenant à la mise en oeuvre de la présente invention sont notamment les suivants :
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

Les compositions cosmétiques selon l'invention peuvent se présenter sous forme de liquides plus ou moins épaissis, de gels, de crèmes, de mousses aérosol.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 4 et 9. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras supérieurs à C₁₆, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions détergentes selon l'invention peuvent bien entendu contenir en outre tous les adjuvants usuels rencontrés dans le domaine des shampooings, comme par exemple des parfums, des agents conservateurs, des sequestrants, des épaississants, des adoucissants, des modificateurs de mousse, des colorants, des agents hydratants, d'autres agents anti-pelliculaires ou anti-séborrhéiques, des filtres solaires et autres.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou amphotères ou cationiques, les protéines, les hydrolysats de protéines, les cires naturelles ou synthétiques, les céramides, les pseudocéramides, les acides gras à chaînes linéaires ou ramifiées en C₁₆-C₄₀ tels que l'acide méthyl-18 eicosanoique, les esters gras d'acides carboxyliques, les mono-, di- ou triglycérides d'acides gras, les vitamines, les provitamines telles que le panthénol, les silicones, volatiles ou non volatiles, solubles et insolubles dans le milieu, les huiles végétales, les huiles de synthèses telles que les poly-alphaoléfines, les huiles fluorées ou perfluorées, et leurs mélanges.

Les compositions selon l'invention comprennent de préférence au moins un polymère cationique et/ou une silicone.

Parmi les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères, en particulier les polymères ou copolymères de chlorure de diméthyldiallylammonium et d'acrylamide, vendus sous les dénominations « MERQUAT 100 », « MERQUAT 550 » et « MERQUAT S » par la Société CALGON, les polysaccharides cationiques tels que les gommes de guar modifiées par du chlorure de 2,3-époxypropyl triméthylammonium commercialisées par exemple sous la dénomination « JAGUAR C13S » par la société MEYHALL et les copolymères vinylpyrrolidone / méthacrylamidopropyl diméthylamine commercialisés notamment sous la dénomination STYLEZE CC 10 par la société ISP et leurs mélanges.

Selon l'invention, le ou les polymères cationiques peuvent représenter de 0,01 % à 10 % en poids, de préférence de 0,05 % à 5 % en poids, et encore plus préférentiellement de 0,1 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Les additifs sont généralement présents dans les compositions selon l'invention en une proportion comprise entre 0,01 % et 20 % en poids, et de préférence entre 0,02 % et 10 % en poids par rapport au poids total de la composition.

Les compositions selon l'invention sont utilisées de préférence comme shampooings pour le lavage et le traitement des cheveux et du cuir chevelu et ils sont appliqués, dans ce cas-là, sur des cheveux humides ou secs dans des quantités efficaces pour les laver, cette application étant suivie d'un rinçage de préférence à l'eau, après un éventuel temps de pose.

La présente invention a également pour objet un procédé de traitement cosmétique comprenant l'application sur les cheveux ou le cuir chevelu d'une composition telle que définie précédemment cette application étant suivie d'un rinçage de préférence à l'eau, après un éventuel temps de pose.

Les exemples qui suivent illustrent la présente invention sans toutefois présenter un caractère limitatif.

### EXEMPLE 1

On a préparé un shampooing de composition suivante

| | |
|---|---|
| Lauryléther sulfate de sodium (2,2 OE) | 12,6 g MA |
| N-cocoyl amidoéthyl, N-éthoxycarboxyméthyl glycinate de sodium (MIRANOL C2M CONC NP de RHODIA CHIMIE) | 0,76 g MA |
| Cocoylbétaïne (DEHYTON AB30 de GOLDSCHMIDT) | 1,2 g MA |
| Pyrithione de Zinc en suspension aqueuse à 48% de MA | 0,96 g MA |
| Acide citrique | 3 g |
| Hydroxyéthylcellulose réticulée par l'épichlorhydrine et quaternisée par de la triéthylamine (JR400 de UNION CARBIDE) | 0,25 g |
| Distéarate d'éthylèneglycol | 2 g |
| Acide polyacrylique réticulé | 0,3 gMA |
| Conservateurs, parfum | q. s |
| pH ajusté à 5,5 | 5,5 |
| Eau q. s. p | 100 g |

Après avoir humidifié les cheveux, on applique une quantité suffisante du shampooing puis on fait mousser et laisse pauser environ 2 min. On rince ensuite abondamment la chevelure. Ce shampooing utilisé régulièrement permet d'éliminer et de prévenir la réapparition des pellicules.

### Détermination de l'activité fongistatique

L'activité fongistatique des compositions A à D a été déterminée par une méthode classique dite de CMI (Concentration Minimale Inhibitrice), la CMI correspondant à la concentration la plus faible à laquelle un produit donné inhibe la croissance d'une souche donnée dans des conditions définies.
En l'occurrence, des souches de Malassezia ovalis (ou Pityrosporum ovale) CIP 1363.82 ont été utilisées.
La détermination de l'activité fongistatique des produits a donc été réalisée par la méthode des dilutions en milieu gélosé
100 µl de produit ont été ajoutés à 1.9 ml de milieu de culture gélosé en surfusion à 45°C.
Des dilutions successives, en progression géométrique de raison 2, de la suspension obtenue ont été réalisées à l'aide du milieu de culture gélosé en surfusion dans les puits d'une plaque (Falcon, 24 puits). Après solidification du milieu, 4µl de la suspension microbienne ont été déposés en surface à l'aide d'une micropipette.
Après 48 heures d'incubation à 30°C, la concentration minimale inhibitrice (C.M.I.) était donnée par la plus faible concentration de produit qui inhibait la croissance microbienne.
La concentration de l'agent antimicrobien à partir de laquelle la croissance du micro-organisme est complètement inhibée (absence de trouble du milieu) est ainsi déterminée et exprimée en dilution.
Les résultats sont rassemblés dans le tableau suivant :

| | A Invention | B | C | D Invention |
|---|---|---|---|---|
| Pyrithione de Zn | 0,96 gMA | 0,96 gMA | ― | 0,96 gMA |
| Acide citrique | 3 g | 3 g | - | 3 g |
| Cocoylbétaïne (DEHYTHON AB 30) | 13,8 gMA | ― | 1,2 g MA | 1,2 g MA |
| APG (KAG 40 de KAO) | | | | |
| Lauryléther sulfate de sodium (2,2 OE) | - | 13,8 gMA | 12,6 gMA | 12,6 gMA |
| Sulfosuccinate de Na | ― | ― | | ― |
| Eau qsp | 100 g | 100 g | 100 g | 100 g |
| CMI | 1/2560 | 1/1280 | 1/80 | 1/2560 |

Plus la CMI est faible, plus la composition est efficace.

## Revendications

1. Composition cosmétique de lavage et de traitement antipelliculaire des cheveux et du cuir chevelu, **caractérisée par le fait qu'**elle contient dans un milieu aqueux :
a) au moins un agent antipelliculaire choisi parmi les sels de pyridinethione
b) au moins un agent tensioactif choisi parmi les tensioactifs amphotères appartenant au groupe des bétaines;
c) au moins 1% en poids d'au moins un hydroxyacide.

2. Composition selon la revendication 1, **caractérisée par le fait que** les sels de pyridinethione sont choisis parmi les sels de calcium, de magnésium, de barium, de strontium, de zinc, de cadmium, d'étain et de zirconium.

3. Composition selon la revendication 1 ou 2, **caractérisée par le fait que** le sel de pyridinethione est le sel de zinc.

4. Composition selon l'une quelconque des revendications 1 à 3, **caractérisée par le fait que** ledit agent antipelliculaire est présent dans des proportions allant de 0,1 à 5% en poids par rapport au poids total de la composition.

5. Composition selon l'une quelconque des revendications précédentes, **caractérisée par le fait que** l'hydroxyacide est présent sous forme d'acide libre et/ou sous la forme de l'un de ses sels associés et/ou sous la forme du lactide correspondant.

6. Composition selon l'une quelconque des revendications précédentes, **caractérisée par le fait que** l'hydroxyacide est choisi parmi les acides citrique, lactique, méthyllactique, phényllactique, malique, mandélique, glycolique, tartronique, tartrique, gluconique, benzylique et 2-hydroxycaprylique.

7. Composition selon l'une quelconque des revendications 1 à 6, **caractérisée par le fait que** ledit hydroxy acide est présent dans des proportions allant de 2 à 10% en poids par rapport au poids total de la composition.

8. Composition selon l'une quelconques des revendications 1 à 7, **caractérisée par le fait que** le tensioactif amphotère est choisi parmi les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

9. Composition selon l'une quelconque des revendications 1 à 9, **caractérisée par le fait que** le(s) agent(s) tensioactif(s) amphotère(s) sont présents à raison de 0,5 à 15% en poids par rapport au poids total de la composition.

10. Composition selon l'une quelconque des revendications 1 à 9, **caractérisée par le fait qu'**elle comprend en outre au moins un agent tensioactif anionique.

11. Composition selon la revendication 10, **caractérisée par le fait que** ledit tensioactif est présent en une quantité comprise entre 0,1% et 40% en poids environ, de préférence entre 3% et 30% par rapport au poids total de la composition.

12. Composition selon l'une quelconque des revendications précédente, **caractérisée par le fait qu'**elle comprend en outre un additif choisi parmi les agents tensioactifs cationiques, les polymères anioniques ou non ioniques ou amphotères ou cationiques, les protéines, les hydrolysats de protéines, les cires naturelles ou synthétiques, les céramides, les pseudocéramides, les acides gras à chaînes linéaires ou ramifiées en C₁₆-C₄₀ tels que l'acide méthyl-18 eicosanoique, les esters gras d'acides carboxyliques, les mono-, di- ou triglycérides d'acides gras, les vitamines, les provitamines telles que le panthénol, les silicones, volatiles ou non volatiles, solubles et insolubles dans le milieu, les huiles végétales, les huiles de synthèses telles que les poly-alphaoléfines, les huiles fluorées ou perfluorées, et leurs mélanges.

13. Composition selon la revendication précédente, **caractérisée par le fait que** ledit polymère cationique est choisi parmi les dérivés d'éther de cellulose quaternaires, les cyclopolymères, les polysaccharides cationiques, les copolymères vinylpyrrolidone méthacrylamidopropyl dimethylamine et leurs mélanges.

14. Composition selon l'une quelconque des revendications 12 ou 13, **caractérisée par le fait que** ledit polymère cationique est présent à une teneur pondérale comprise entre 0,01 % et 10 % par rapport au poids total de la composition et de préférence comprise entre 0,05 % et 5 %.

15. Composition selon l'une quelconque des revendications précédentes, **caractérisée par le fait qu'**elle présente un pH compris entre 4 et 9.

16. Utilisation d'une composition telle définie à l'une quelconque des revendications précédentes pour le lavage et le traitement antipelliculaire des cheveux et du cuir chevelu.

17. Procédé de traitement cosmétique ou dermatologique comprenant l'application sur les cheveux ou le cuir chevelu d'une composition telle que définie précédemment, cette application étant suivie d'un rinçage de préférence à l'eau, après un éventuel temps de pose.
